(19) Europäisches Patentamt
European Patent Office
Office européen des brevets

(11) Veröffentlichungsnummer: **0 029 160**
**A1**

(12) **EUROPÄISCHE PATENTANMELDUNG**

(21) Anmeldenummer: **80106767.9**

(22) Anmeldetag: **04.11.80**

(51) Int. Cl.³: **C 07 D 249/18**, C 07 D 249/22, C 07 D 249/16, A 01 N 43/64

(30) Priorität: **13.11.79 DE 2945689**

(43) Veröffentlichungstag der Anmeldung: **27.05.81 Patentblatt 81/21**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Mues, Volker, Dr., Gellertweg 13, D-5600 Wuppertal 1 (DE)**
Erfinder: **Behrenz, Wolfgang, Dr., Untergruendemich 14, D-5063 Overath (DE)**

(54) **Neue Oxybenztriazolderivate, Verfahren zu ihrer Herstellung und ihre Verwendung als Synergisten in Schädlingsbekämpfungsmitteln.**

(57) Die Erfindung betrifft neue Oxybenztriazolderivate der Formel

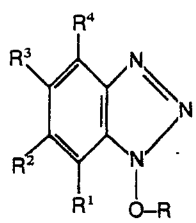

(I)

gefunden, in welcher

R für Alkenyl oder Alkinyl steht

und die Reste

$R^1$, $R^2$, $R^3$ und $R^4$, welche gleich oder verschieden sein können, für Wasserstoff, Cyano, Nitro, Halogen, Carbamoyl, Monoalkylamino- oder Dialkylamino-carbonyl, Aminosulfonyl, Monoalkylamino- oder Dialkylaminosulfonyl, für gegebenenfalls durch Halogen, Cyano, Alkoxy oder Alkylthio substituiertes Alkyl, oder für gegebenenfalls halogen-substituiertes Alkoxy, Alkylthio oder Alkylsulfonyl stehen, oder in welcher zwei benachbarte Reste $R^1$ und $R^2$, $R^2$ und $R^3$ oder $R^3$ und $R^4$ zusammen für Alkylen oder Benzo stehen,

ein Verfahren zu ihrer Herstellung aus Hydroxybenztriazolen und Halogen-alkenen bzw. Halogen-alkinen und ihre Verwendung als Synergisten in Schädlingsbekämpfungsmitteln, insbesondere insktiziden und akariziden Mitteln.

EP 0 029 160 A1

BAYER AKTIENGESELLSCHAFT          5090 Leverkusen, Bayerwerk

Zentralbereich
Patente, Marken und Lizenzen    S-by

# Neue Oxybenztriazolderivate, Verfahren zu ihrer Herstellung und ihre Verwendung als Synergisten in Schädlingsbekämpfungsmitteln

Die Erfindung betrifft neue Oxybenztriazolderivate, ein Verfahren zu ihrer Herstellung und ihre Verwendung als Synergisten in Schädlingsbekämpfungsmitteln.

Es ist bereits bekannt, daß die folgenden Wirkstoffe bzw. Wirkstoffgruppen pestizide, insbesondere insektizide und akarizide Eigenschaften besitzen:

A) Carbaminsäureester, wie z.B. N-Methyl-O-(2-iso-propoxy-phenyl)-carbaminsäureester, N-Methyl-O-(2,3-dihydro-2,2-dimethyl-7-benzofuranyl)-carbaminsäureester, N-Methyl-O-(2,3-(dimethyl-methylendioxy)-phenyl)-carbaminsäureester und N-Methyl-O-(1-methylthio-ethyliden-amino)-carbaminsäureester;

B) Carbonsäureester, wie z.B. 2,2-Dimethyl-3-(2-methyl-propen-1-yl)-cyclopropancarbonsäure-(2,3,4,5-tetra-

hydro-phthalimido-methyl)-ester, 2,2-Dimethyl-3-(2,2-dichlor-vinyl)-cyclopropancarbonsäure-(3-phenoxy-benzyl)-ester, 2,2-Dimethyl-3-(2,2-dichlor-vinyl)-cyclopropancarbonsäure-(α-cyano-4-fluor-3-phenoxy-benzyl)-ester, 2,2-Dimethyl-3-(2,2-dichlor-vinyl)-cyclopropancarbonsäure-(pentafluor-benzyl)-ester, 2,2-Dimethyl-3-(2,2-dibrom-vinyl)-cyclopropancarbonsäure-(α-cyano-3-phenoxy-benzyl)-ester, 3-Methyl-2-(4-chlor-phenyl)-butansäure-(α-cyano-3-phenoxy-benzyl)-ester, die natürlich vorkommenden Pyrethroide und Essigsäure-(α-trichlormethyl-3,4-dichlor-benzyl)-ester;

C) Halogen(cyclo)alkane, wie z.B. Hexachlorcyclohexan, sowie

D) Phosphorsäure- und Phosphonsäureester.

Weiterhin sind synergistische Mischungen von Carbaminsäureestern, wie z.B. 2-Isopropoxy-phenyl-N-methylcarbamat oder von Phosphorsäureestern, z.B. O,O-Diethyl-O-(2-isopropyl-4-methyl-pyrimidin(6)yl)-thionophosphorsäureester oder von natürlichen oder synthetischen Pyrethroiden mit Piperonylethern, wie z.B. α-(2-(2-Butoxy-ethoxy)-ethoxy)-4,5-methylendioxy-2-propyl-toluol, bekannt (vgl. Bull. Wld. Health Org. 1966, 35, Seiten 691-708; Schrader, G.: Die Entwicklung neuer insektizider Phosphorsäureester 1963, S. 158; Perkov, W.: Die Insektizide, 1966, Seiten 516-524). Doch ist die Wirksamkeit dieser synergistischen Wirkstoffkombinationen nicht befriedigend. Eine gewisse praktische Bedeutung hat bisher nur das α-(2-(2-Butoxy-ethoxy)-ethoxy)-4,5-methylendioxy-2-propyl-toluol erlangt.

Es wurden nun die neuen Oxybenztriazolderivate der Formel I

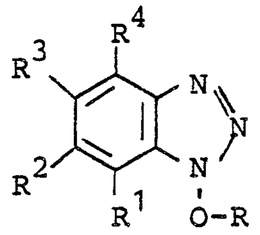

(I)

gefunden, in welcher

R für Alkenyl oder Alkinyl steht und die Reste

$R^1$,$R^2$,$R^3$ und $R^4$, welche gleich oder verschieden sein können, für Wasserstoff, Cyano, Nitro, Halogen, Carbamoyl, Monoalkylamino - oder Dialkylaminocarbonyl, Aminosulfonyl, Monoalkylamino- oder Dialkylaminosulfonyl, für gegebenenfalls durch Halogen, Cyano, Alkoxy oder Alkylthio substituiertes Alkyl, oder für gegebenenfalls halogensubstituiertes Alkoxy, Alkylthio oder Alkylsulfonyl stehen, oder in welcher zwei benachbarte Reste $R^1$ und $R^2$, $R^2$ und $R^3$ oder $R^3$ und $R^4$ zusammen für Alkylen oder Benzo stehen.

Die neuen Oxybenztriazolderivate sind durch die Formel (I) definiert. Vorzugsweise stehen darin

R für $C_3$-$C_6$-Alkenyl oder $C_3$-$C_5$-Alkinyl und die Reste

$R^1$ bis $R^4$ für Wasserstoff, Chlor, Brom, Nitro, Cyano, Methyl, Methoxy, Ethoxy, Methylthio, Trifluormethyl, Trifluormethoxy, Bis-(trifluormethyl)-amino oder trifluormethylthio.

Man erhält Verbindungen der Formel (I), wenn man Hydroxybenztriazole der Formel (II)

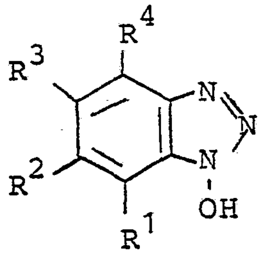

(II).

in welcher

$R^1$, $R^2$, $R^3$ und $R^4$ die oben angegebenen Bedeutungen haben,

mit Halogen-alkenen bzw. Halogen-alkinen der Formel (III)

X-R (III)

in welcher

X für Chlor, Brom oder Jod und

R für Alkenyl oder Alkinyl steht,

gegebenenfalls in Gegenwart eines Säureakzeptors und gegebenenfalls unter Verwendung eines Verdünnungsmittels umsetzt.

Verwendet man als Ausgangsstoffe beispielsweise 1-Hydroxybenztriazol und Propargylbromid, so kann die Reaktion dieser Verbindungen durch folgendes Formelschema skizziert werden:

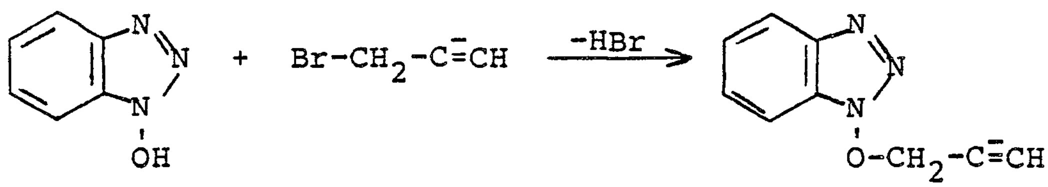

Die als Ausgangsstoffe zu verwendenden Verbindungen sind durch die Formeln (II) und (III) definiert. Vorzugsweise haben darin die Reste R bis $R^6$ die gleichen Bedeutungen, wie sie bei der vorzugsweisen Definition der entsprechenden Reste in Formel (I) genannt sind.

Die Ausgangsstoffe der Formeln (II) und (III) sind bekannt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methyl-isobutylketon, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 150°C, vorzugsweise bei 20 bis 100°C.

Das Herstellungsverfahren wird im allgemeinen bei Normaldruck durchgeführt.

Zur Durchführung des erfindungsgemäßen Verfahrens werden die Ausgangsstoffe gewöhnlich in angenähert äquimolaren Mengen eingesetzt. Ein Überschuß der einen oder anderen Reaktionskomponenten bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man ein organisches Lösungsmittel, z.B. Methylenchlorid oder Toluol zu, filtriert und destilliert das Filtrat.

Kombinationen aus den neuen Oxybenztriazolderivaten der Formel (I) und

A) Carbaminsäureestern,
B) Carbonsäureestern,

0029160

C) Halogen(cyclo)alkanen und/oder

D) Phosphorsäure- und Phosphonsäureestern, die ein weiterer Gegenstand der Erfindung sind,

zeichnen sich durch hohe pestizide, insbesondere insektizide und akarizide Wirksamkeit aus.

Die Verbindungen der Formel (I) zeigen synergistische Wirkung vorzugsweise bei

A) Carbaminsäureestern der Formel (IV)

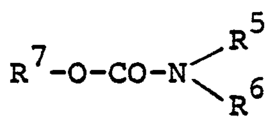

(IV)

in welcher

$R^5$ für Wasserstoff oder $C_1$-$C_4$-Alkyl steht,

$R^6$ für $C_1$-$C_4$-Alkyl, für gegebenenfalls durch Halogen, Hydroxy oder Methylthio substituiertes $C_1$-$C_4$-Alkylcarbonyl oder für den Rest S-Z steht, wobei

Z für gegebenenfalls halogen-substituiertes $C_1$-$C_4$-Alkyl oder für gegebenenfalls durch Halogen, Cyano, $C_1$-$C_4$-Alkyl, Trihalogenmethylthio, Trihalogenmethyl und/oder Nitro substituiertes Phenyl, oder für $C_1$-$C_4$-Alkoxy-carbonyl steht, und

$R^7$ für Aryl, für einen heterocyclischen Rest oder für einen Oximrest steht.

Als Wirkstoffkomponenten ganz besonders bevorzugt sind Carbaminsäureester der Formel (IV), in welcher

$R^7$ für gegebenenfalls durch $C_1$-$C_4$-Alkyl, $C_1$-$C_4$-Alkoxy, $C_1$-$C_4$-Alkoxy-methyl, $C_1$-$C_4$-Alkylthio, $C_1$-$C_4$-Alkylthio-methyl, $C_1$-$C_4$-Alkylamino, Di-($C_1$-$C_4$-alkyl)-amino, Di-($C_2$-$C_4$-alkenyl)-amino und/oder Halogen substituierte Reste Phenyl, Naphthyl, 2,3-Dihydrobenzofuranyl, Methylendioxyphenyl, Dioxolanylphenyl, Pyrazolyl oder Pyrimidinyl steht, oder in welcher

$R^7$ für einen Alkylidenamino-rest der Formel (IVa)

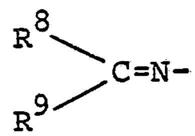

(IVa)

steht, in welcher

$R^8$ und $R^9$ gleich oder verschieden sind und einzeln für $C_1$-$C_5$-Alkyl, $C_3$-$C_8$-Cycloalkyl, $C_2$-$C_5$-Alkenyl, $C_2$-$C_5$-Alkinyl, $C_1$-$C_4$-Alkoxy, $C_1$-$C_4$-Alkylthio, $C_1$-$C_4$-Alkylthio-methyl, $C_1$-$C_4$-Alkoxycarbonyl, Carbamoyl, Cyano oder Phenyl, oder zusammen für gegebenenfalls durch $C_1$-$C_4$-Alkyl substituiertes Alkylendioxy oder Alkylendimercapto stehen.

Als Beispiele für die Carbaminsäureester der Formel (IV) seien genannt:
2-Methyl-phenyl-, 2-Ethyl-phenyl-, 2-iso-Propyl-phenyl-, 2-sek.-Butyl-phenyl-, 2-Methoxy-phenyl-, 2-Ethoxy-phenyl-, 2-iso-Propoxy-phenyl-, 4-Methyl-phenyl-, 4-Ethyl-phenyl-, 4-n-Propyl-phenyl-, 4-Methoxy-phenyl-, 4-Ethoxy-phenyl-, 4-n-Propoxy-phenyl-, 3,4,5-Trimethyl-phenyl-, 3,5-Dimethyl-4-methylthio-phenyl-, 3-Methyl-4-dimethylaminophenyl-, 2-Ethylthiomethyl-phenyl-, 1-Naphthyl-, 2,3-Dihydro-2,2-dimethyl-7-benzofuranyl-, 2,3-(Dimethyl-methylen-dioxy)-phenyl-, 2-(4,5-Dimethyl-1,3-dioxolan-2-yl)-phenyl-, 1-Methylthio-ethyliden-amino-, 2-Methylthio-2-methyl-propyliden-amino-, 1-(2-Cyano-ethylthio-ethyliden-amino- und 1-Methylthiomethyl-2,2-dimethyl-propyliden-amino-N-methyl-carbaminsäureester.

Weiter zeigt sich die synergistische Wirkung der neuen Verbindungen der Formel (I) vorzugsweise bei

B) Carbonsäureestern der Formel (V)

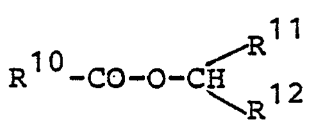

(V)

in welcher

$R^{10}$ für einen offenkettigen oder cyclischen Alkylrest steht, der gegebenenfalls substituiert ist durch Halogen, Alkyl, Cycloalkyl, durch gegebenenfalls durch Halogen und/oder Alkoxy substituiertes Alkenyl, durch Phenyl oder Styryl, welche gegebenenfalls durch Halogen, gegebenenfalls halogen-substituierte Reste Alkyl, Alkoxy, Alkylendioxy und/oder Alkylthio substituiert sind, durch spirocyclisch verknüpftes, gegebenenfalls halogen-substituiertes Cycloalk(en)yl, welches gegebenenfalls benzannelliert ist, in welcher weiter

$R^{11}$ für Wasserstoff, Alkyl, Halogenalkyl, Alkenyl, Alkinyl oder Cyano steht, und

$R^{12}$ für einen gegebenenfalls substituierten Arylrest oder für einen Heterocyclus steht, oder zusammen mit $R^{11}$ und dem Kohlenstoffatom, an das beide Reste gebunden sind, einen Cyclopentenonring bildet.

Als Wirkstoffkomponenten ganz besonders bevorzugt sind Carbonsäureester der Formel (V), in welcher

$R^{10}$ für den Rest

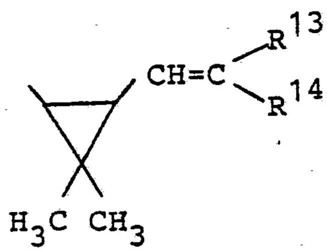

steht, worin

$R^{13}$ für Wasserstoff, Methyl, Fluor, Chlor oder Brom und

$R^{14}$ für Methyl, Fluor, Chlor, Brom, $C_1$-$C_2$-Fluoralkyl oder $C_1$-$C_2$-Chlorfluoralkyl oder für gegebenenfalls durch Halogen und/oder gegebenenfalls halogen-substituierte Reste $C_1$-$C_4$-Alkyl, $C_1$-$C_4$-Alkoxy, $C_1$-$C_4$-Alkylthio und/oder $C_1$-$C_2$-Alkylendioxy substituiertes Phenyl steht oder worin beide Reste $R^{13}$ und $R^{14}$ für $C_2$-$C_5$-Alkandiyl (Alkylen) stehen; oder in welcher

$R^{10}$ für den Rest $-\underset{R^{16}}{\underset{|}{CH}}-R^{15}$

steht, worin

$R^{15}$ für gegebenenfalls durch Halogen und/oder durch gegebenenfalls halogen-substituierte Reste $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^{16}$ für Isopropyl oder Cyclopropyl steht;

oder in welcher

$R^{10}$ für einen der Reste

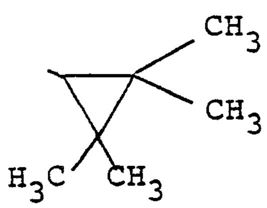
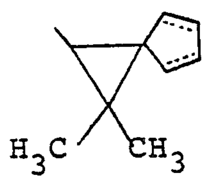
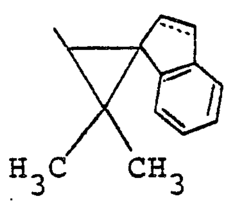
oder Methyl

steht; in welcher weiter

$R^{11}$ für Wasserstoff, $C_1$-$C_4$-Alkyl, $C_1$-$C_4$-Halogenalkyl, Cyano oder Ethinyl steht und

$R^{12}$ für gegebenenfalls durch Halogen und/oder durch einen gegebenenfalls halogen-substituierten Rest $C_1$-$C_4$-Alkyl, $C_2$-$C_4$-Alkenyl, $C_1$-$C_4$-Alkoxy, $C_2$-$C_4$-Alkenoxy, $C_1$-$C_4$-Alkylthio, $C_1$-$C_2$-Alkylendioxy, Phenoxy und/oder Benzyl substituierte Reste Phenyl, Furyl oder Tetrahydrophthalimido steht.

Weiter sind die natürlich vorkommenden Pyrethroide besonders bevorzugt.

Als Beispiele für die Carbonsäureester der Formel V seien genannt: Essigsäure-(2,2,2-Trichlor-1-(3,4-dichlor-phenyl)-ethyl)-ester, 2,2-Dimethyl-3-(2-methyl-propen-1-yl)-cyclopropan-carbonsäure-(2,3,4,5-tetrahydro-phthalimido-methyl)-ester, 2,2-Dimethyl-3-(2,2-dichlor-vinyl)-cyclopropancarbonsäure-(3-phenoxy-benzyl)-ester, 2,2-Dimethyl-3-(2,2-dichlor-vinyl)-cyclopropan-carbonsäure-(α-cyano-4-fluor-3-phenoxy-benzyl)-ester, 2,2-Dimethyl-3-(2,2-dichlor-vinyl)-cyclopropancarbonsäure-(pentafluor-benzyl)-ester, 2,2-Dimethyl-3-(2,2-dibrom-vinyl)-cyclopropan-carbonsäure-(α-cyano-3-phenoxy-benzyl)-ester, 3-Methyl-2-(4-chlor-phenyl)-butansäure-(α-cyano-3-phenoxy-benzyl)-ester und 2,2-dimethyl-3-(2,2-dichlorvinyl)-cyclopropancarbonsäure-α-cyano-(4-fluor-3-phenoxy)-benzylester.

C) Die synergistische Wirkung der Verbindung der Formel (I) zeigt sich ferner vorzugsweise bei Halogen(cyclo)-alkanen, wie z.B. Hexachlorcyclohexanen, 1,1,1-Trichlor-2,2-bis-(4-chlor-phenyl)-ethan, 1,1,1-Trichlor-2,2-bis-(4-methoxy-phenyl)-ethan und 1,1-Dichlor-2,2-bis-(4-ethyl-phenyl)-ethan.

D) Bevorzugte Phosphorsäure- und Phosphonsäureester D haben die Formel

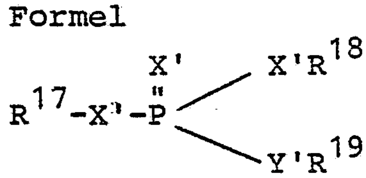

(VI)

in welcher

X' für Sauerstoff oder Schwefel steht,

Y' für Sauerstoff, Schwefel, Imino oder für eine direkte Bindung zwischen dem P-Atom und $R^1$ steht,

$R^{17}$ und $R^{18}$ gleich oder verschieden sind und für gegebenenfalls substituiertes Alkyl oder Aryl stehen

und

$R^{19}$ für gegebenenfalls substituierte Reste Alkyl,Aryl, Heteroaryl, Aralkyl, Alkenyl, oder einen Oximrest oder für den gleichen Rest, an den es gebunden ist steht.

Besonders bevorzugt sind hierbei Phosphorsäure- und Phosphonsäureester der Formel (VI), in welcher

$R^{17}$ und $R^{18}$ gleich oder verschiecen sind und für $C_1$-$C_4$-Alkyl oder Phenyl stehen und

$R^{19}$ für gegebenenfalls durch Halogen, Hydroxy und/oder Cyano substituiertes $C_1$-$C_4$-Alkyl, für gegebenenfalls durch Halogen, Cyano, Nitro, Carbamoyl, $C_1$-$C_4$-Alkyl-sulfinyl, $C_1$-$C_4$-Alkylthio-carbonyl, $C_1$-$C_4$-Alkyl, $C_1$-$C_4$-Alkoxy, $C_1$-$C_4$-Alkylthio und/oder $C_1$-$C_4$-Alkoxy-carbonyl substituiertes Phenyl, für gegebenenfalls durch Halogen und/oder halogensubstituiertes Phenyl, $C_1$-$C_4$-Alkoxy-carbonyl, $C_1$-$C_4$-Alkyl-amino-carbonyl oder Di-($C_1$-$C_4$-alkyl)-aminocarbonyl substituiertes $C_2$-$C_4$-Alkenyl oder für einen Rest der Formel IVa

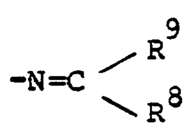 (IVa)

steht, worin $R^9$ und $R^8$ die oben angegebenen Bedeutungen haben, wobei jedoch insbesondere $R^9$ für Cyano und $R^8$ für $C_1$-$C_4$-Alkyl oder Phenyl steht;

$R^{19}$ steht ferner insbesondere für gegebenenfalls durch Halogen, $C_1$-$C_4$-Alkyl, $C_1$-$C_4$-Alkoxy, $C_1$-$C_4$-Alkylthio, $C_1$-$C_4$-Alkyl-amino, Di-($C_1$-$C_4$-alkyl)-amino, $C_1$-$C_4$-Alkoxymethyl, $C_1$-$C_4$-Alkylthiomethyl, $C_1$-$C_4$-Alkyl-sulfinylmethyl und/oder $C_1$-$C_4$-Alkyl-sulfonylmethyl substituierte heteroaromatische Reste wie Pyridinyl, Chinolinyl, Chinoxalinyl, Pyrimidinyl, Benzo-oxotriazinyl, Pyrazolyl oder Imidazolyl.

Als Beispiel für die Phosphorsäureester der Formel (VI) sei O,O-Dimethyl-O-(2,2-dichlor-vinyl)-phosphorsäureester genannt.

Überraschenderweise ist die pestizide Wirksamkeit der erfindungsgemäßen Wirkstoffkombinationen wesentlich höher als die Wirkung der Einzelkomponenten bzw. die Summe der Wirkungen der Einzelkomponenten. Sie ist ferner wesentlich höher als die Wirkung von Wirkstoffkombinationen mit dem bekannten Synergisten Piperonylbutoxid. Ferner haben die erfindungsgemäßen Verbindungen ein wesentlich breiteres synergistisches Wirkungspotenzial als andere bereits bekannt gewordene Synergisten.

Die Gewichtsverhältnisse der Wirkstoffgruppen können dabei in relativ großen Bereichen schwanken. Im allgemeinen werden die als Synergisten verwendeten Verbindungen mit den übrigen Wirkstoffen im Verhältnis 0,1:10 bis 10:0,1 eingesetzt. Besonders vorteilhaft sind jedoch Mischungsverhältnisse von 0,5:1,0 bis 1,0:1,0. Die erfindungsgemäßen Wirkstoffkombinationen bewirken nicht nur eine schnelle knock-down-Wirkung, sondern bewirken auch die nachhaltige Abtötung der tierischen Schädlinge, insbesondere von Insekten und Milben, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Die Wirkstoffkombinationen eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 2 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

0029160

## Herstellungsbeispiele

### Beispiel 1

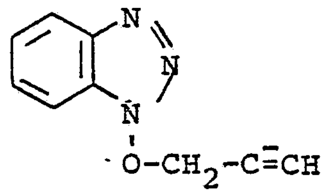

Eine Mischung von 30,6 g (0,2 Mol) 1-Hydroxybenztriazolhydrat, 26,2 g (0,22 Mol) Propargylbromid, 30,4 g (0,22 Mol) Kaliumcarbonat und 500 ml Acetonitril wird unter Rühren 20 Stunden zum Sieden unter Rückfluß erhitzt. Nach dem Abkühlen auf Raumtemperatur wird das Reaktionsgemisch mit Methylenchlorid verdünnt und abgesaugt. Das Filtrat wird unter vermindertem Druck destilliert. Man erhält 30,0 g (86,7 % der Theorie) 1-Prop-2-inoxy-benztriazol als gelbes Öl vom Siedepunkt 122°C/2 mbar.

### Beispiel 2

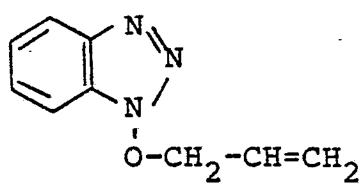

Aus 30,6 g (0,2 Mol) 1-Hydroxybenztriazol, 26,6 g (0,22 Mol) Allylbromid und 30,4 g (0,22 Mol) Kaliumcarbonat erhält man analog Beispiel 1 31,5 g (90 % der Theorie) 1-Prop-2-enoxy-benztriazol als gelbes Öl vom Siedepunkt 126°C/6 mbar.

Gemäß den Beispielen 1 und 2 werden die folgenden Verbindungen erhalten:

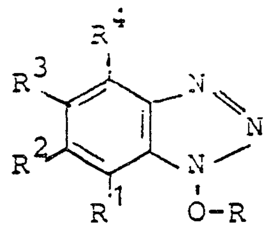

| Beispiel Nr. | R | $R^1$ | $R^2$ | $R^3$ | $R^4$ | Fp. °C / Brechungsindex $n_{25}^{D}$ |
|---|---|---|---|---|---|---|
| 3 | $-CH_2-CH=CH_2$ | H | Cl | H | H | 54 |
| 4 | $-CH_2-C\equiv CH$ | H | Cl | H | H | 98 |
| 5 | $-CH_2-C\equiv CH$ | H | H | H | Cl | 76 - 79 |
| 6 | $-CH_2-C\equiv CH$ | H | H | Cl | H | 102 |
| 7 | $-CH_2-CH=CH_2$ | H | H | Cl | H | 1,5704 |
| 8 | $-CH_2-CH=CH_2$ | H | H | H | Cl | 1,5718 |
| 9 | $-CH_2-C\equiv CH$ | H | Cl | Cl | H | 112 |
| 10 | $-CH_2-CH=CH_2$ | H | Cl | Cl | H | 63 |
| 11 | $-CH_2-CH=CH_2$ | H | $CF_3$ | H | H | 1,4990 |
| 12 | $-CH_2-C\equiv CH$ | H | $CF_3$ | H | H | 83 |
| 13 | $-CH_2-CH=CH_2$ | H | $CH_3$ | H | H | 1,5565 |
| 14 | $-CH_2-C\equiv CH$ | H | $CH_3$ | H | H | 53 |
| 15 | $-CH_2-C\equiv CH$ | H | $CH_3$ | Cl | H | 102 - 104 |
| 16 | $-CH_2-C\equiv CH$ | H | H | Cl | Cl | 119 |
| 17 | $-CH_2-C\equiv CH$ | H | Br | H | H | 139 |
| 18 | $-CH_2-C\equiv CH$ | H | $OCH_3$ | H | H | 105 |
| 19 | $-CH_2-C\equiv CH$ | H | Cl | Cl | Cl | 155 |
| 20 | $-CH_2-C\equiv CH$ | H | Cl | H | Cl | 135 |
| 21 | $-CH_2-C\equiv CH$ | H | Cl | H | $CF_3$ | 101 |
| 22 | $-CH_2-C\equiv CH$ | H | $SCF_3$ | H | H | 79 |

| Beispiel Nr. | R | $R^1$ | $R^2$ | $R^3$ | $R^4$ | Fp. °C / Brechungsindex $n_{25}^{D}$ |
|---|---|---|---|---|---|---|
| 23 | $-CH_2-C\equiv CH$ | H | $CF_3$ | Cl | H | 64 |
| 24 | $-CH_2-C\equiv CH$ | H | $CF_3$ | H | Cl | 97 |
| 25 | $-CH_2-C\equiv CH$ | H | $CF_3$ | H | $CF_3$ | 71 |
| 26 | $-CH_2-C\equiv CH$ | H | $N(CF_3)_2$ | H | H | Öl |

Beispiel A

$LT_{100}$-Test

Testtiere: Musca domestica (gegen Phosphorsäureester resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipetiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Testtiere wird bis zu 6 Stunden laufend kontrolliert. Es wird diejenige Zeit ermittelt, die für eine 100 %ige knock down-Wirkung erforderlich ist. Wird die $LT_{100}$ nach 6 Stunden nicht erreicht, wird der % Satz der knock down gegangenen Testtiere festgestellt.

Konzentrationen der Wirkstoffe, Synergisten und Gemische und ihre Wirkungen gehen aus der nachfolgenden Tabelle hervor.

LT 100-Test mit phosphorsäureester-resistenten Musca domestica (Stamm Weymanns)

| Wirkstoff ()Kennbuchstabe | bzw. Synergist () Beispiel Nr. | Konzentration in % Wirkstoff | Synergist | LT 100 n.Min. |
|---|---|---|---|---|
| 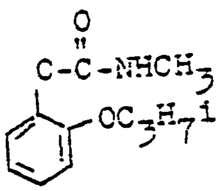<br>(A) | | 1,0 | | 360' = 60 % |
| 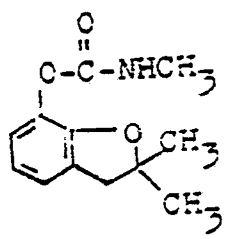<br>(B) | | 0,04 | | 360' = 70 % |
| 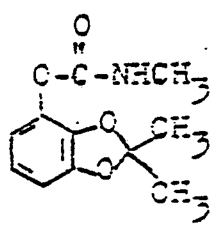<br>(C) | | 0,2 | | 360' = 80 % |
| 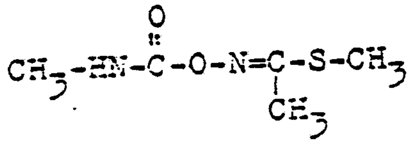<br>(D) | | 0,04 | | 150' |
| Pyrethrine als 25 %iger Extrakt<br>(E) | | 0,04 | | 360' = 50 % |

| Wirkstoff () Kennbuchstabe | bzw. Synergist () Beispiel Nr. | Konzentrationen in % Wirkstoff | Konzentrationen in % Synergist | LT 100 n.Min. |
| --- | --- | --- | --- | --- |
| 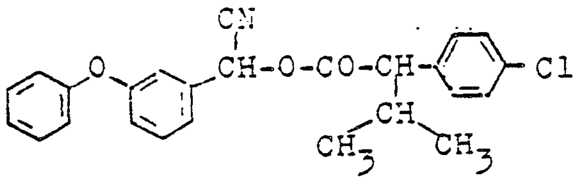 (F) | | 0,008 | | 180' |
| 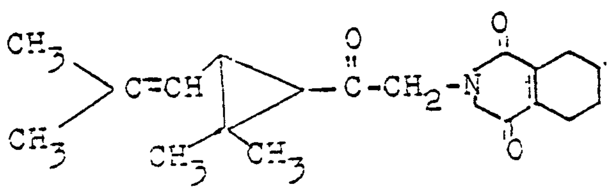 (G) | | 0,04 | | 150' |
| 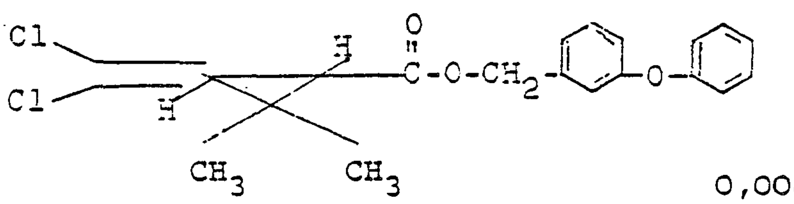 (H) | | 0,008 | | 210' |
| 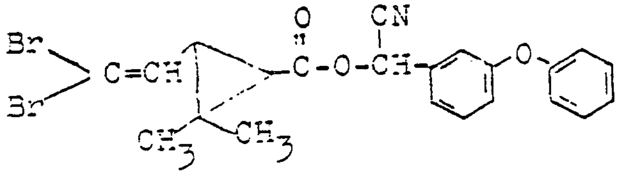 (I) | | 0,008 | | 75' |

| Wirkstoff () Kennbuchstabe | bzw. Synergist () Beispiel Nr. | Konzentrationen in % Wirkstoff | Synergist | LT 100 n.Min. |
|---|---|---|---|---|
| 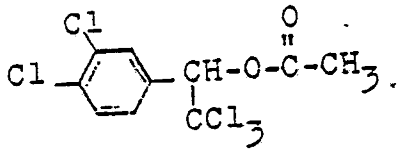 (K) | | 0,2 | | 360'= 70 % |
| 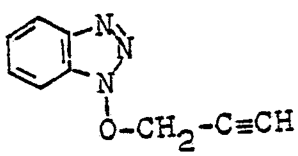 (1) | | 0,2 | | 360' |
| 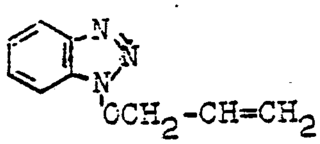 (2) | | 0,2 | | 180' |
| 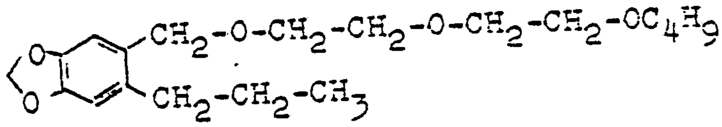 (3) Piperonylbutoxid (bekannt) | | 0,2 | | 360'=0 % |

| Wirkstoff () Kennbuchstabe | bzw. Synergist () Beispiel Nr. | Konzentrationen in % Wirkstoff Synergist | LT 100 n.Min. |
|---|---|---|---|
| A + 3 | | 0,2 + 0,2 | 360' = 30 % |
| A + 1 | | 0,008 + 0,008 | 180' |
| A + 2 | | 0,04 + 0,04 | 120' |
| B + 3 | | 0,2 + 0,2 | 360' = 95 % |
| B + 1 | | 0,008 + 0,008 | 210' |
| B + 2 | | 0,04 + 0,04 | 105' |
| C + 3 | | 0,2 + 0,2 | 360' = 95 % |
| C + 1 | | 0,04 + 0,04 | 120' |
| C + 2 | | 0,2 + 0,2 | 90' |
| D + 3 | | 0,008 + 0,008 | 360' |
| D + 1 | | 0,008 + 0,008 | 210' |
| D + 2 | | 0,008 + 0,008 | 240' |
| E + 1 | | 0,04 + 0,04 | 60' |
| E + 2 | | 0,04 + 0,04 | 45' |
| F + 3 | | 0,008 + 0,008 | 150' |
| F + 2 | | 0,008 + 0,008 | 120' |
| G + 3 | | 0,04 + 0,04 | 105' |
| G + 1 | | 0,04 + 0,04 | 60' |
| G + 2 | | 0,04 + 0,04 | 45' |
| H + 3 | | 0,008 + 0,008 | 150' |
| H + 2 | | 0,008 + 0,008 | 105' |
| I + 3 | | 0,008 + 0,008 | 60' |
| I + 2 | | 0,008 + 0,008 | 45' |
| K + 3 | | 0,2 + 0,2 | 360' = 80 % |
| K + 1 | | 0,008 + 0,008 | 180' |
| K + 2 | | 0,04 + 0,04 | 120' |

Patentansprüche

1) Oxybenztriazolderivate der Formel

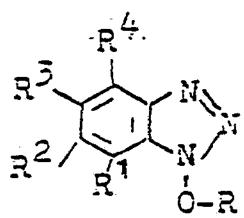

(I)

in welcher

R für Alkenyl oder Alkinyl steht und die Reste

$R^1$, $R^2$, $R^3$ und $R^4$, welche gleich oder verschieden sein können, für Wasserstoff, Cyano, Nitro, Halogen, Carbamoyl, Monoalkylamino- oder Dialkylamino-carbonyl, Aminosulfonyl, Monoalkylamino- oder Dialkylaminosulfonyl, für gegebenenfalls durch Halogen, Cyano, Alkoxy oder Alkylthio substituiertes Alkyl, oder für gegebenenfalls halogen-substituiertes Alkoxy, Alkylthio oder Alkylsulfonyl stehen, oder in welcher zwei benachbarte Reste $R^1$ und $R^2$, $R^2$ und $R^3$ oder $R^3$ und $R^4$ zusammen für Alkylen oder Benzo stehen.

2) Verfahren zur Herstellung von Verbindungen der Formel (I), dadurch gekennzeichnet, daß man Hydroxybenztriazole der Formel II

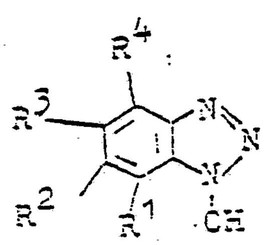

(II)

in welcher
$R^1$, $R^2$, $R^3$ und $R^4$ die in Anspruch 1 angegebene Bedeutung haben,

mit Halogen-alkenen oder Halogen-alkinen der Formel III

$$X-R \qquad (III)$$

in welcher
X für Chlor, Brom oder Jod und
R für Alkenyl oder Alkinyl steht,

gegebenenfalls in Gegenwart eines Säureakzeptors und gegebenenfalls unter Verwendung eines Verdünnungsmittels umsetzt.

3) Schädlingsbekämpfungsmittel, gekennzeichnet durch einen Gehalt an einer Wirkstoffkombination bestehend aus den Oxybenztriazolderivaten gemäß Anspruch 1 und

A) Carbaminsäureestern,
B) Carbonsäureestern,
C) Halogen(cyclo)alkanen und/oder
D) Phosphorsäure- und Phosphonsäureestern.

4) Verwendung von Oxybenztriazolderivaten gemäß Anspruch 1 als Synergisten in Schädlingsbekämpfungsmitteln.

5) Verfahren zur Bekämpfung von Schädlingen, insbesondere von Insekten und Spinnentieren, dadurch gekennzeichnet, daß man eine Wirkstoffkombination gemäß Anspruch 3 auf Schädlinge, insbesondere auf Insekten und Spinnentiere und/oder ihren Lebensraum einwirken läßt.

6) Verwendung von Wirkstoffkombinationen gemäß Anspruch 3 zur Bekämpfung von Schädlingen, insbesondere von Insekten und Spinnentieren.

7) Verfahren zur Herstellung von Schädlingsbekämpfungsmitteln, insbesondere insektiziden und akariziden Mitteln, dadurch gekennzeichnet, daß man eine Wirkstoffkombination gemäß Anspruch 3 mit Streckmitteln und/oder oberflächenaktiven Mitteln vermischt.

Europäisches Patentamt

# EUROPÄISCHER RECHERCHENBERICHT

0029160

Nummer der Anmeldung

EP 80 10 6767

| Kategorie | EINSCHLÄGIGE DOKUMENTE<br>Kennzeichnung des Dokuments mit Angabe, soweit erforderlich, der maßgeblichen Teile | betrifft Anspruch |
|---|---|---|
| | US - A - 4 038 403 (D.P. WRIGHT)<br>* Spalten 1-6 *<br>-- | 1-7 |
| P | US - A - 4 174 285 (M. BRAID)<br>* Spalten 1-6 *<br>---- | 1 |

KLASSIFIKATION DER ANMELDUNG (Int. Cl.³)

C 07 D 249/18
249/22
249/16
A 01 N 43/64

RECHERCHIERTE SACHGEBIETE (Int. Cl.³)

C 07 D 249/18

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 29-01-1981 | CREMERS |